# EUROPEAN PATENT APPLICATION

(11) **EP 2 949 321 A1**
(43) Date of publication of application: **02.12.2015**
(21) Application number: 15169075.7
(22) Date of filing: 25.05.2015
(51) Int. Cl.: A61K 9/24, A61K 31/47, A61K 31/445

(54) **MULTILAYER FORMULATIONS OF FEXOFENADINE AND MONTELUKAST**

(30) Priority: 26.05.2014 TR 201405875
(71) Applicant: Sanovel Ilac Sanayi ve Ticaret A.S., 34460 Istanbul (TR)
(72) Inventor: CIFTER, Ümit, 34460 Istanbul (TR); TÜRKYILMAZ, Ali, 34460 Istanbul (TR); PEHLIVAN AKALIN, Nur, 34460 Istanbul (TR); TUNA, Sevda, 34460 Istanbul (TR)
(74) Representative: Sevinç, Erkan

(57) **Abstract**

The present invention relates to a multilayer tablet formulation comprising fexofenadine hydrochloride and montelukast sodium.

## Description

### Field of Invention

The present invention relates to a multilayer tablet formulation comprising fexofenadine hydrochloride and montelukast sodium.

### Background of Invention

Fexofenadine hydrochloride is a non-sedating antihistamine pharmaceutical active agent used in the treatment of the allergy symptoms of seasonal allergic rhinitis including runny nose, sneezing or watery eyes; or itching of the nose, throat, or roof of the mouth. The chemical name of fexofenadine hydrochloride is 2-[4-[(1RS)-1-hydroxy-4-[4-(hydroxydiphenylmethyl)piperidin-1-yl]butyl] phenyl]-2-methylpropanoic acid hydrochloride and has the structure shown in the following formula I. Fexofenadine hydrochloride can exist in four polymorphic forms, Form I, Form II, Form III and form IV crystals.

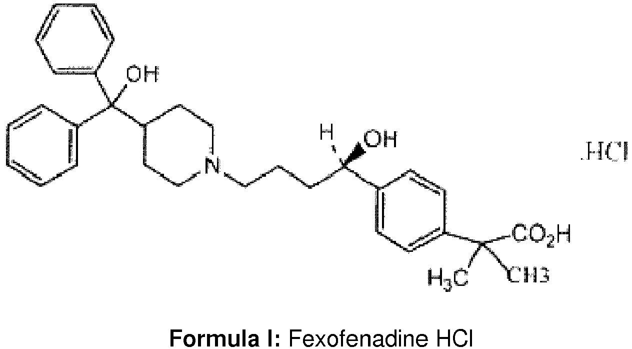

In prior art, fexofenadine hydrochloride is first disclosed in patent numbered US 4,254,129. Fexofenadine hydrochloride also exists in the market in tablet, ODT (orally disintegrating tablet) and oral suspension forms under the brand name of ALLEGRA^{®} in U.S.

Montelukast sodium has one asymmetric center and is the R-isomer. A crystalline and an amorphous form are known. Montelukast sodium is a leukotriene receptor antagonist approved for the prophylaxis and chronic treatment of asthma. The chemical name of montelukast is [R-(E)]-1-[[[1-[3-[2-(7-chloro-2-quinolinyl)ethenyl]phenyl]-3-[2-(1-hydroxy-1-methylethyl)-phenyl]propyl]thio]methyl] cyclopropane acetic acid mono sodium salt and has the structure shown in the following formula II. It works by blocking the action of substances in the body that cause the symptoms of asthma and allergic rhinitis. It is used to prevent wheezing, difficulty breathing, chest tightness, and coughing caused by asthma.

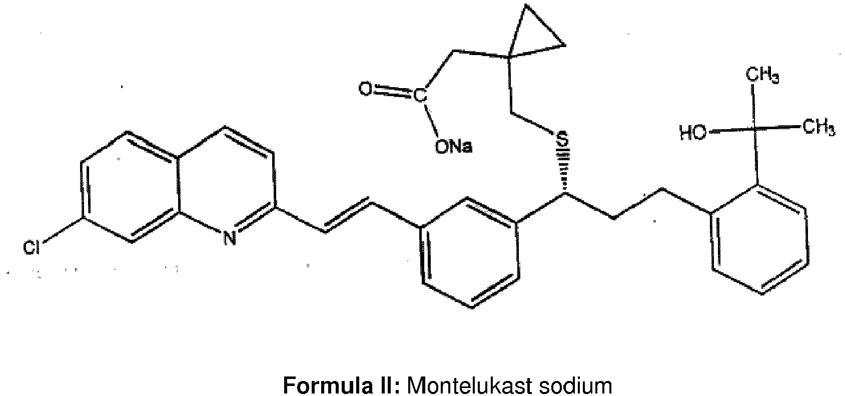

In patent WO9716173, tablet formulation of montelukast sodium has been diclosed. It also exists in the market as tablet, chewable tablet and oral granule under the brand name of SINGULAIR^{®} in U.S.

Fexofenadine hydrochloride as a second generation antihistaminic has more advantageous than first generation antihistaminic. Because it cannot pass through the blood-brain barrier, it causes less sedation and other side effects as well as having longer plasma half-life. On the other side, montelukast is also widely used drug as an add-on therapy for allergic rhinitis.

The risk of asthma for patients who has allergic rhinitis is much more than healthy people. Leukotrienes are responsible from the symptoms of allergic rhinitis. Therefore, in the case of control of symptoms cannot be provided sufficiently, it is expected to provide better allergic rhinitis control when second generation H₁ - antihistamine fexofenadine HCl with montelukast which is a leukotriene receptor antagonist are used together. It has been also found that the combination of fexofenadine and montelukast is more effective compared to fexofenadine alone in the control of allergic rhinitis symptoms in clinical trials.

In addition, instead of one per se use, combining more than one molecule in one dosage form increases the patients' quality of life and patients' compliance. Considering all these, combinations of fexofenadine and montelukast in a suitable pharmaceutical dosage formulation is needed. However, there are many challenges while combining two or more molecules in a tablet such as compatibility, dissolution and stability problems.

In this invention, to overcome problems in tablet formulations of fexofenadine montelukast combination, a multilayer tablet formulation has been developed. In further, in order to overcome stability problem, wet granulation of montelukast with ethanol has been provided.

### Description of the invention

The main embodiment of the present invention is to provide a multilayer tablet formulation comprising fexofenadine hydrochloride and montelukast sodium.

In one embodiment, the pharmaceutical combination is in the form of a multilayer tablet. According to one embodiment of the present invention is to provide a pharmaceutical combination wherein fexofenadine or a pharmaceutically acceptable salt is present in an amount of 10.00 - 50.00 % by weight of total formulation and preferably it is 20.00 - 35.00 % by weight of total formulation and montelukast or a pharmaceutically acceptable salt is present in an amount of 1.00 - 30.00 % by weight of total formulation and preferably it is 3.00 - 15.00 % by weight of total formulation

One embodiment of this present invention is to obtain a multilayer tablet formulation comprising a stable and compatible combination of fexofenadine and montelukast with a desired dissolution.

In one embodiment, the multilayer tablet formulation comprises three layers; a fexofenadine layer, a montelukast layer and an inert layer separating these two layers from each other.

According to another embodiment of this invention, the layer comprising fexofenadine and the layer comprising montelukast are separated using an inert layer to prevent an interaction, therefore the incompatibility problem of these agents is eliminated and efficient and desired dissolution are obtained.

In order to combine two different molecules in one dosage form, molecules should be compatible with each other to achieve desired stability and dissolution for the patients' compliance. During the development study to combine fexofenadine and montelukast, it has been found that montelukast shows stability and dissolution problems when they used in a conventional tablet formulation (one layer). The analysis results of combination in conventional tablets shows an increase in impurity results and a decrease in dissolution of montelukast. Owing to the incompatibility problem of these active agents, in this invention multilayer tablet has been developed and desired dissolution and stability has been achieved.

According to one embodiment, due to cellulosic polymers in the inert layer, the efficient and desired dissolution of both active agents are achieved rapidly. Cellulosic polymers give advantages to formulation in terms of binding strength to other two layers. With the synergistic effect of cellulosic polymers used in inert layer and layers comprising active miolecules, layers adhere to each other and robust multilayer tablet with desired dissolution has been achieved. Cellulosic polymers used in this present invention are microcrystalline cellulose, hydroxypropyl cellulose, hydroxypropyl ethyl cellulose, hydroxypropyl methyl cellulose, preferably it is microcrystalline cellulose.

According to this embodiment, the amount of microcrystalline cellulose in inert layer is between 20.00 - 95.00 % by weight of total inert layer and preferably it is 70.00 - 95.0 % by weight of total inert layer. The amount of microcrystalline cellulose in montelukast layer is between 30.00 - 80.00 % by weight of montelukast layer and preferably it is 40.00 - 50.0 % by weight of montelukast layer and the amount of microcrystalline cellulose in fexofenadine layer is between 30.00 - 80.00 % by weight of fexofenadine layer and preferably it is 40.00 - 50.0 % by weight of fexofenadine layer.

According to this embodiment, dissolution test of multilayer tablet has been performed by pellet method in 900 mL sodium 0.5% solution of sodium dodecyl sulphate and at 37 °C ± 0.5 °C at 75 rpm.

In one embodiment, the granulation solution of the montelukast layer is ethanol.

In one embodiement, in prior art, montelukast is granulated with water. In contrast, in this present invention, to further improve the stability of multilayer tablet, ethanol (96%) is used as a granulation solution for layer comprising montelukast. It has been found that, when ethanol has been used during the wet granulation, amount of impurity has been decreased.

According to this embodiment, stability analysis of montelukast has been performed by gradient reverse phase HPLC (high performance liquid chromatography) method at 236 nm UV light and at 35 °C column temperature.

Suitable fillers may include, but not limited to lactose, starch, prejelatinized starch, microcrystalline cellulose, mannitol, dibasic calcium phosphate, tribasic calcium phosphate, trehalose, isomalt, sodium carbonate, sodium bicarbonate, calcium carbonate or mixtures thereof.

Suitable disintegrants may include, but not limited to croscarmellose sodium, polyvinylpyrrolidone (K - 30), povidone, HPC (hydroxylpropyl cellulose) (LH 11, LF), HPMC (hydroxylpropyl methyl cellulose), carboxy methyl cellulose calcium, alginic acid and alginates, ion-exchange resins, magnesium aluminum silica, sodium dodecyl sulphate, sodium carboxy methyl cellulose, crospovidone, docusate sodium, guar gam, polyacrylin potasium, poloxomer, sodium alginate, sodium glysin carbonate, sodium lauryl sulphate., sodium starch glycolate, soy polysaccharide, gellan gum, xanthan gum, calcium silicate or mixtures thereof.

Suitable binders may include but not limited to microcrystalline cellulose (PH 101, PH 102), polyvinylpyrrolidone, sugars, glycose syrups, natural gums, guar gum, gelatins, pullulan, agar, alginate, sodium algynates, K-Karagen, glycyrrhizin, polymetacrylates, kollagen, agar, algynate, sodium alginate, hyaluronic acid, pectin, tragakanti gum, carboxymethyl cellulose, polyethylene glycol, polyvinyl alcohol, polyvinyl acetate and their copolymers, cellulose derivatives such as hydroxypropyl methyl cellulose, carboxy methyl cellulose, methyl cellulose, polyvinylalcohol, carrageenan, carbomer, poloxamer, polyacrylamide, aluminum hydroxide, benthonite, laponite, setostearyl alcohol, polyoxyethylene-alkyl ethers, acacia mucilage, polydextrose, polyethylene oxide, xylitol, sucrose stearate, or mixtures thereof.

Suitable lubricants may include but not limited to magnesium stearate, sodium stearyl fumarate, polyethylene glycol, sodium lauryl sulphate, magnesium lauryl sulphate, fumaric acid, glyceryl palmitostearate, hydrogenated natural oils, zinc stearate, calcium stearate, silica, talc, stearic acid, polyethylene glycol, paraffin or mixtures thereof.

Suitable glidants may include but not limited to colloidal silicon dioxide, aluminium silicate or mixtures thereof.

Coating may also preferably be used for moisture protection. It can be selected from the group comprising iron oxide yellow, polyvinyl alcohol-polyethylene glycol copolymers (Kollicoat IR), polyvinyl alcohol or copolymers or mixtures thereof (Opadry AMB), Ethylcellulose Dispersions (Surelease), Kerry-HPC, polyethylene glycol, polyvinylprolidone, polyvinylprolidone-vinyl acetate copolymer(PVP-VA) and all kinds of OpadryTM, as well as pigments, dyes, titanium dioxide, talc and polymethylmetacrylate copolymers (Eudragit).

### Example 1:

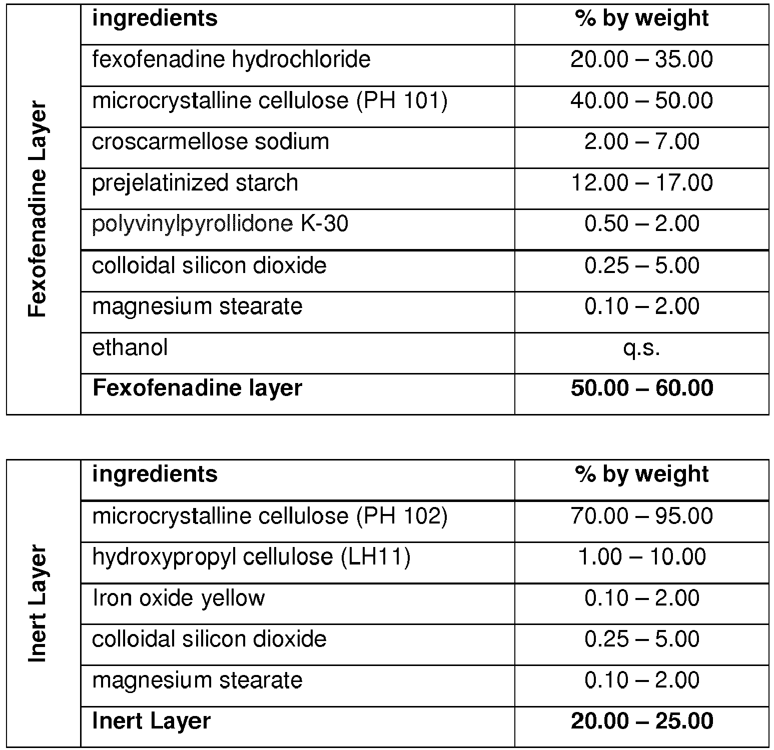

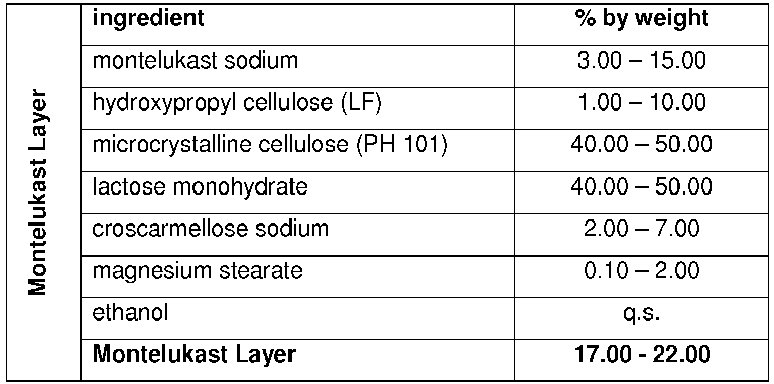

The production of the formulation is carried out as follows:
**Fexofenadine layer:** fexofenadine hydrochloride, microcrystalline cellulose (PH 101), croscarmellose sodium, prejelatinized starch and polyvinylpyrollidone K-30 are taken into collete and powder mixture is granulated with ethanol. The mixture is sieved and dried then sieved again. Colloidal silicon dioxide is added to this mixture and mixed. Then, magnesium stareate was added and mixed again.
**Inert Layer:** A part of microcrystalline cellulose (PH 102) and hydroxypropyl cellulose (LH11) are taken into a container. Iron oxide yellow, colloidal silicon dioxide and other part of microcrystalline cellulose are sieved and taken into container. Powder mixture is mixed and magnesium stearate is added to this mixture. Total mixture is sieved and mixed.
**Montelukast Layer:** montelukast sodium, hydroxypropyl cellulose (LF), microcrystalline cellulose (PH 101), lactose monohydrate and croscarmellose sodium are taken into collete. Powder mixture is wet granulated with ethanol. The mixture is sieved and dried then sieved again. Magnesium stearate is added to this mixture and mixed.

All homogenous mixtures for each layer are pressed into multilayer tablet comprising between 50.00 - 60.00 % of fexofenadine layer, 20.00 - 25.00 of inert layer and 17.00 - 22.00 % of montelukast layer by weight of total formulation.

## Claims

1. A multilayer tablet formulation comprising fexofenadine hydrochloride and montelukast sodium.

2. The multilayer tablet formulation according to claim 1, wherein the multilayer tablet formulation comprises three layers.

3. The multilayer tablet formulation according to claim 1 or 2, wherein the three layers are fexofenadine layer, inert layer and montelukast layer.

4. The multilayer tablet formulation according to claim 3, wherein the inert layer comprises cellulosic polymers.

5. The multilayer tablet formulation according to claim 4, wherein the cellulosic polymers are microcrystalline cellulose, hydroxypropyl cellulose, hydroxypropyl ethyl cellulose, hydroxypropyl methyl cellulose or mixtures thereof, preferably it is microcrystalline cellulose.

6. The multilayer tablet formulation according to claim 5, wherein the amount of microcrystalline cellulose in inert layer is between 20.00 - 95.00 % by weight of total inert layer and preferably it is 70.00 - 95.0 % by weight of total inert layer.

7. The multilayer tablet formulation according to any preceding claim, wherein The amount of microcrystalline cellulose in montelukast layer is between 30.00 - 80.00 % by weight of montelukast layer and preferably it is 40.00 - 50.0 % by weight of montelukast layer and the amount of microcrystalline cellulose in fexofenadine layer is between 30.00 - 80.00 % by weight of fexofenadine layer and preferably it is 40.00 - 50.0 % by weight of fexofenadine layer.

8. The multilayer tablet formulation according to any preceding claim, wherein the granulation solution of the montelukast layer is ethanol.
